# EUROPÄISCHE PATENTSCHRIFT

(11) **EP 2 839 824 B1**
(45) Veröffentlichungstag und Bekanntmachung des Hinweises auf die Patenterteilung: **25.10.2017**
(21) Anmeldenummer: 14171068.1
(22) Anmeldetag: 04.06.2014
(51) Int. Cl.: A61K 8/06, A61Q 17/04, A61K 8/29, A61K 8/31, A61K 8/37, A61K 8/49

(54) **Sonnenschutz mit hohem Triazingehalt**
Sun protection with high triazine content
Protection solaire à teneur en triazine élevée

(30) Priorität: 04.07.2013 DE 102013213175
(43) Veröffentlichungstag der Anmeldung: 25.02.2015
(73) Patentinhaber: Beiersdorf AG, 20253 Hamburg (DE)
(72) Erfinder: Skubsch, Kerstin, 25497 Prisdorf (DE); Nielsen, Jens, 22529 Hamburg (DE)

(56) Entgegenhaltungen:
- WO-A2-2008/014505
- DE-A1-102005 059 742
- US-A1- 2012 128 611
- Anonymous: "GNPD - Baby Sun Block SPF 100", , 1. April 2014 (2014-04-01), Seiten 1-2, XP055164554, Internet Gefunden im Internet: URL:http://www.gnpd.com/sinatra/recordpage /2368645/from_search/3AeX1Hghjr/ [gefunden am 2015-01-23]

## Beschreibung

Die vorliegende Erfindung betrifft eine kosmetische Zubereitung enthaltend 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoesäure-tris(2-ethylhexylester) und/oder Diethylhexyl Butamido Triazin in Kombination mit 2-Phenylethylbenzoat und Di-n-butyladipat.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Insbesondere um hohe Lichtschutzfaktoren zu erreichen ist es nahezu unumgänglich Triazinderivate als UV-Filter einzusetzen. Diese Klasse der UV-Filter hat jedoch den

EM 2013-004 Octocrylenfreie Sonnenschutzmittel X-Tend 226 und Butap mit Triazinen bekannten Nachteil, nur schwer in der Ölphase der Zubereitungen löslich zu sein. Dies gilt insbesondere für die UV-Filter) 2,4-Bis-{[4-(2-ethyl-hexyloxy)-2-hydroxy]-phenyl}-6-(4-methoxyphenyl)-1,3,5-triazin) und 4,4',4"-(1,3,5-Triazin-2,4,6-triyitriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone) und Diethylhexyl Butamido Triazin.

Im Stande der Technik finden sich eine Vielzahl an Publikationen, die sich dem Löslichkeitsproblem der Triazinderivate annehmen. Besonders attraktiv ist dabei zunächst einmal die Kombination mit bei Raumtemperatur flüssigen, lipophilen UV-Filtern wie Ethylhexyl-2-cyano-3,3-diphenylacrylat (Octocrylen) aber auch Homomenthylsalicylat, 4-Methoxyzimtsäure(2-ethylhexyl)ester, Ethylhexylsalicylat und Kampferderivate wie 3-(4-Methylbenzyliden)campher. So beschreibt beispielsweise die EP 1034778 die Verwendung von Octocrylen zur Solubilisierung des 2,4-Bis-{[4-(2-ethyl-hexyloxy)-2-hydroxy]-phenyl}-6-(4-methoxyphenyl)-1,3,5-triazin.

Der Nachteil des Standes der Technik besteht nun in dem Umstand, dass der Einsatz dieser UV-Filter, trotz ihrer Zulassung durch die Genehmigungsbehörden, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass diese UV-Filter möglicherweise hormonell wirksam sein könnten. Auch wenn, trotz des jahrzehntelangen weltweiten Einsatzes dieser UV-Filter in Sonnenschutzmitteln keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, ein Triazin-haltiges (insbesondere 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoesäure-tris(2-ethylhexylester) und/oder Diethylhexyl Butamido Triazin, enthaltendes) Sonnenschutzmittel zu entwickeln, in welchen diese UV-Filter langzeit- und temperaturstabil gelöst vorliegen, ohne dass hierfür auf die bekannten, bei Raumtemperatur flüssigen UV-Filter zurückgegriffen werden muss..

Überraschend gelöst wird die Aufgabe durch eine kosmetische Zubereitung enthaltend
a) 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoësäure-tris(2-ethylhexylester) und/oder Dioctylbutylamidotriazon (INCI: Diethylhexyl-Butamidotriazone)
b) 2-Phenylethylbenzoat,
c) Di-n-butyladipat,
dadurch gekennzeichnet, dass die Zubereitung frei ist von Polyethylenglycol, Polyethylenglycolethern und Polyethylenglycolestern.

Dabei ist es erfindungsgemäß besonders bevorzugt, wenn die erfindungsgemäße Zubereitung frei ist von N-Lauroylisopropylsarcosinat, Ethylhexylsalicylat, 2-Ethylhexyl-2-cyano-3,3-diphenylacrylat, Homomenthylsalicylat und 4-Methoxyzimtsäure(2-ethylhexyl)ester und 3-(4-Methylbenzyliden)campher.

Zwar kennt der Stand der Technik die US 2012/128611, DE10 2005 059 742, WO 2008/014505 und Anonymus: "GNPD-Baby Sun Block SPF 100" vom 1.April 2014 (XP055164554), doch konnten diese Offenbarungen nicht den Weg zur vorliegenden Erfindung weisen.

Erfindungsgemäß vorteilhafte Ausführungsformen der vorliegenden Erfindung sind dadurch gekennzeichnet, dass die Zubereitung 2-Phenylethylbenzoat in einer Konzentration von 1 bis 20 Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung 2-Phenylethylbenzoat in einer Konzentration von 2 bis 10 Gewichts-%, bezogen auf das Gesamtgewicht der Zubereitung, enthält.

Es ist vorteilhaft im Sinne der vorliegenden Erfindung, wenn die erfindungsgemäße Zubereitung Di-n-butyladipat in einer Konzentration von 0,5 bis 10 Gewichts-%, bezogen auf das Gesamtgewicht der Zubereitung, enthält.

Es ist bevorzugt im Sinne der vorliegenden Erfindung, wenn die erfindungsgemäße Zubereitung Di-n-butyladipat in einer Konzentration von 1 bis 5 Gewichts-%, bezogen auf das Gesamtgewicht der Zubereitung, enthält.

Darüber hinaus ist es erfindungsgemäß besonders vorteilhaft, wenn das Gewichtsverhältnis von 2-Phenylethylbenzoat zu Di-n-butyladipat von 5 1: bis 1:5 beträgt.

Erfindungsgemäß vorteilhafte Ausführungsformen der vorliegenden Erfindung sind dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoë-säure-tris(2-ethylhexylester) und/oder Diethylhexyl Butamido Triazin, in einer Gesamtmenge von 0,1 bis 15 Gewichts-%, bezogen auf das Gesamtgewicht der Zubereitung, enthält.

Enthält die erfindungsgemäße Zubereitung 2,4-Bis-{[4-(2-ethyl-hexyloxy)-2-hydroxy]-phenyl}-6-(4-methoxyphenyl)-1,3,5-triazin ohne 4,4',4"-(1,3,5-Triazin-2,4,6-triyltriimino)-tris-benzoë-säure-tris(2-ethylhexylester) zu enthalten, so liegt die erfindungsgemäß vorteilhafte Einsatzkonzentration für diesen UV-Filter zwischen 0,1 und 6 Gewichts-%, bezogen auf das Gesamtgewicht der Zubereitung und die erfindungsgemäß bevorzugte Einsatzkonzentration zwischen 0,5 und 4 Gewichts-%, bezogen auf das Gesamtgewicht der Zubereitung.

Enthält die erfindungsgemäße Zubereitung 4,4',4"-(1,3,5-Triazin-2,4,6-triyltriimino)-tris-ben-zoësäure-tris(2-ethylhexylester) ohne 2,4-Bis-{[4-(2-ethyl-hexyloxy)-2-hydroxy]-phenyl}-6-(4-methoxyphenyl)-1,3,5-triazin zu enthalten, so liegt die erfindungsgemäß vorteilhafte Einsatzkonzentration für diesen UV-Filter zwischen 0,1 und 6 Gewichts-%, bezogen auf das Gesamtgewicht der Zubereitung und die erfindungsgemäß bevorzugte Einsatzkonzentration zwischen 0,5 und 4 Gewichts-%, bezogen auf das Gesamtgewicht der Zubereitung.

Enthält die erfindungsgemäße Zubereitung sowohl 2,4-Bis-{[4-(2-ethyl-hexyloxy)-2-hydroxy]-phenyl}-6-(4-methoxyphenyl)-1,3,5-triazin als auch 4,4',4"-(1,3,5-Triazin-2,4,6-triyltriimino)-tris-benzoësäure-tris(2-ethylhexylester), so liegt die erfindungsgemäß vorteilhafte Einsatzkonzentration für 2,4-Bis-{[4-(2-ethyl-hexyloxy)-2-hydroxy]-phenyl}-6-(4-methoxyphenyl)-1,3,5-triazin zwischen 0,1 und 6 Gewichts-% sowie für 4,4',4"-(1,3,5-Triazin-2,4,6-triyltriimino)-tris-benzoësäure-tris(2-ethylhexylester) zwischen 0,1 und 6 Gewichts-%, jeweils bezogen auf das Gesamtgewicht der Zubereitung.

Die erfindungsgemäß bevorzugte Einsatzkonzentration beträgt in einem solchen Falle für 2,4-Bis-{[4-(2-ethyl-hexyloxy)-2-hydroxy]-phenyl}-6-(4-methoxyphenyl)-1,3,5-triazin von 0,5 bis 4 Gewichts-% und für 4,4',4"-(1,3,5-Triazin-2,4,6-triyltriimino)-tris-benzoësäure-tris(2-ethylhexylester) von 0,5 bis 4 Gewichts-%, jeweils bezogen auf das Gesamtgewicht der Zubereitung.

Erfindungsgemäß vorteilhafte Ausführungsformen der vorliegenden Erfindung sind dadurch gekennzeichnet, dass die Zusammensetzung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Terephthalidendicampher-sulfonsäure; 4-(Dimethylamino)-benzoesäure(2-ethylhexyl)ester; 4-(Dimethylamino)-benzoesäureamylester; 4-Methoxybenzalmalon-säuredi(2-ethylhexyl)-ester; 4-Methoxyzimtsäureisoamylester; 2-(4'-Diethylamino-2'-hydoxybenzoyl)-benzoesäurehexylester, 2-Ethylhexyl-2-hydroxy-benzoat; Dimethicodiethylbenzalmalonat; 4-(tert.-Butyl)-4'-methoxydibenzoylmethan; 3-(4-(2,2-bis Ethoxycarbonylvinyl)-phe-noxy)propenyl)-methoxysiloxan / Dimethylsiloxan - Copolymer; Dioctylbutylamido-triazon (INCI: Diethylhexyl-Butamidotriazone); 2,4-bis-[5-1(dimethylpropyl)benz-oxazol-2-yl-(4-phenyl)-imino]-6-(2-ethylhexyl)-imino-1,3,5-triazin mit der (CAS Nr. 288254-16-0); Zinkoxid, Titandioxid, 2,4,6-Tribiphenyl-4-yl-1,3,5-triazin; Merocyanine

Dabei ist es erfindungsgemäß bevorzugt, wenn die erfindungsgemäße Zubereitung 4-(tert.-Butyl)-4'-methoxydibenzoylmethan enthält.

Es ist erfindungsgemäß, wenn die erfindungsgemäße Zubereitung frei ist von Polyethylenglycol, Polyethylenglycolethern und Polyethylenglycolestern (sogenannten PEG-Derivaten).

Es ist erfindungsgmäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, , Tocopherol, Tocopherolacetat, Glycyrrhiza Inflata Root Extract enthält.

Außerdem sind erfindungsgemäß vorteilhafte Ausführungsformen der vorliegenden Erfindung dadurch gekennzeichnet, dass die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Erfindungsgemäß vorteilhafte Ausführungsformen der vorliegenden Erfindung sind dadurch gekennzeichnet, dass die Zubereitung frei ist von Parabenen, Phenoxyethanol, Benzethoniumchlorid, Piroctone Olamine, Lauroylarginat, Benzoesäure, Sorbinsäure, Methylisothiazolinon, Chlormethylisothiazolinon, Bronopolm Benzalkoniumchloride, Formaldehydabspalter, Salicylsäure, Triclosan, Dehydroacetsäure , DMDM Hydantoin, Chlorphenesin, IPBC.

Vielmehr ist es erfindungsgemäß vorteilhaft, wenn die erfindungsgemäße Zubereitung Ethanol enthält.

Es ist erfindungsgemäß vorteilhaft, wenn die erfindungsgemäße Zubereitung in Form einer Emulsion vorliegt und erfindungsgemäß bevorzugt, wenn die erfindungsgemäße Zubereitung in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate Natriumstearylglutamat, enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 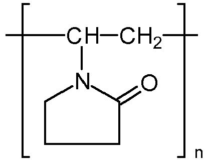 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist die Verwendung der erfindungsgemäßen Zubereitung zum Schutz vor Hautalterung (insbesondere zum Schutz vor UV-bedingter Hautalterung) sowie als Sonnenschutzmittel.

### Löslichkeitsversuche

Es wurden UV-Filterlösungen der folgenden Öle hergestellt und deren Lager- und Temperaturstabilität untersucht. Alle Prozentangaben beziehen sich auf Gewichts-% (Gew.-%).

Die Zubereitungen enthielten
10 Gew.% 2,4-Bis-{[4-(2-ethyl-hexyloxy)-2-hydroxy]-phenyl}-6-(4-methoxyphenyl)-1,3,5-triazin
10 Gew.-% 4,4',4"-(1,3,5-Triazin-2,4,6-triyltriimino)-tris-benzoësäure-tris(2-ethylhexylester)
10 Gew.-% 4-(tert.-Butyl)-4'-methoxydibenzoylmethan

| | 1 Woche | 1 Woche | 4 Wochen | 4 Wochen | 4 Wochen |
|---|---|---|---|---|---|
| | -10°C | +6°C | +6°C | SCHT | -10° |
| (1) 70% Butylene Glycol Dicaprylate/Dicaprate | klar | klar | einzelne Ausfällungen | klar | klar |
| (2) 70% C12-15 Alkyl Benzoate | klar | klar | trübe | klar | trübe |
| (3) 70% Dibutyl Adipate | klar | leichter BS am Rand | deutlicher BS | klar | deutlicher BS |
| (4) 70% Di-C12-13 Alkyl Tartrate | klar | klar | leichter BS | klar | klar |
| (5) 70% Diisopropyl Sebacate | klar | einzelne Flusen | Bodensatz ringförmig | klar | leichter BS |
| (6) 35% 2-Phenylethylbenzoat u. 35% Dibutyl Adipate | klar | klar | klar | klar | klar |
| (7) 35% 2-Phenylethylbenzoat u. 35% C12-15 Alkyl Benzoate | klar | klar | leichter BS | klar | klar |
| (8)23,33% 2-Phenylethylbenzoat, 23,33% , Butylene Glycol Dicaprylate/Dicaprate 23,33% Diisopropyl Sebacate | leichter Bodensatz (BS) | Bodensatz+Flus en | deutlicher BS | klar | deutlicher BS |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **1** | **2** | **3** | **4** | **5** | **6** |
|---|---|---|---|---|---|---|
| Glyceryl Stearat SE | 1 | 1 | 1 | 1 | | |
| Natrium Cetearyl Sulfat | 0,15 | 0,15 | | 0,15 | | |
| Cetearylalkohol | 1 | 3 | 0,5 | 0,5 | | |
| Natriumstearylglutamate | | | | | 0,3 | 0,3 |
| Glyceryl Stearat | | | | | 1 | 1 |
| Acrylates/C₁₀₋₃₀Alkyl Acrylat Crosspolymer | 0,1 | 0,1 | 0,4 | 0,1 | 0,2 | 0,2 |
| Xanthan Gummi | 0,4 | 0,4 | 0,4 | 0,4 | | |
| 2-Phenylethylbenzoat | 5 | 5 | 8 | 5 | 2 | 3 |
| Di-n-butyladipat. | 3 | 4 | 4 | 5 | 5 | 3 |
| C₁₂₋₁₅ Alkyl Benzoat | 8 | | 3 | 5 | | 3 |
| Butylenglycol Dicaprylat/Dicaprat | | 4 | | 5 | | |
| beschichtetes Titanium Dioxid Primärpartikelgröße 10-80 nm | | | 3 | 3 | 3 | 3 |
| Bis-Ethylhexyloxyphenol Methoxyphenyltriazin | | 3 | 2 | 4 | 2 | 2 |
| Ethylhexyltriazin | 5 | 3 | | 3 | 2 | 2 |
| Diethylhexyl Butamido Triazone | | | 2 | | | |
| Butyl Methoxydibenzoylmethan | | 5 | 4 | 5 | 3 | 3 |
| Phenylbenzimidazol Sulfonsäure | | | | | | 1 |
| 2-(4'-(Diethylamino)-2'-hydoxybenzoyl)-benzoesäurehexylester | 5 | | | | | |
| Phenylen-1,4-bis-(2-benzimidazyl)-3,3'-5,5'-tetrasulfonsäure | | | | 1 | | |
| Vitamin E Acetat | | | 0,5 | | | 0,5 |
| Ethanol | 3 | 3 | 4 | 4 | 3 | 5 |
| Ethylhexylglycerin | 0,5 | 0,3 | 0,2 | 0,5 | | |
| Methylpropandiol | 4 | 4 | 3 | 5 | 4 | 3 |
| Na₂H₂EDTA | 0,1 | 0,1 | 0,15 | 0,05 | 0,1 | 0,1 |
| Phenoxyethanol | | 0,1 | | | | 0,4 |
| Panthenol | | 1 | 1 | | 1 | |
| Neutralisationsmittel (z.B. NaOH) | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Zubereitung enthaltend
a) 2,4-Bis-{[4-(2-ethyl-hexyloxy)-2-hydroxy]-phenyl}-6-(4-methoxyphenyl)-1,3,5-triazin. und/oder 4,4',4"-(1,3,5-Triazin-2,4,6-triyltriimino)-tris-benzoësäure-tris(2-ethylhexylester) und/oder Dioctylbutylamidotriazon (INCI: Diethylhexyl-Butamidotriazone),
b) 2-Phenylethylbenzoat,
c) Di-n-butyladipat,
**dadurch gekennzeichnet, dass** die Zubereitung frei ist von Polyethylenglycol, Polyethylenglycolethern und Polyethylenglycolestern.

2. Kosmetische Zubereitung nach Anspruch 1, **dadurch gekennzeichnet, dass** die Zubereitung frei ist von N-Lauroylisopropylsarcosinat, Ethylhexylsalicylat, 2-Ethylhexyl-2-cyano-3,3-di-phenylacrylat, Homomenthylsalicylat und 4-Methoxyzimtsäure(2-ethylhexyl)ester und 3-(4-Methylbenzyliden)campher.

3. Kosmetische Zubereitung nach einem der vorhergehenden Ansprüche, **dadurch gekennzeichnet, dass** die Zubereitung 2-Phenylethylbenzoat in einer Konzentration von 1 bis 20 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Di-n-butyladipat in einer Konzentration von 0,5 bis 10 Gewichts-%, bezogen auf das Gesamtgewicht der Zubereitung, enthält.

5. Kosmetische Zubereitung nach einem der vorhergehenden Ansprüche, **dadurch gekennzeichnet, dass** das Gewichtsverhältnis von 2-Phenylethylbenzoat zu Di-n-butyladipat von 5 1: bis 1:5 beträgt.

6. Kosmetische Zubereitung nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin und/oder 4,4',4"-(1,3,5-Triazin-2,4,6-triyltriimino)-tris-benzoesäure-tris(2-ethylhexylester) und/oder Diethylhexyl Butamido Triazin, in einer Gesamtmenge von 0,1 bis 15 Gewichts-%, bezogen auf das Gesamtgewicht der Zubereitung, enthält.

7. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, , Tocopherol, Tocopherolacetat, Glycyrrhiza Inflata Root Extract enthält.

8. Kosmetische Zubereitung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

9. Kosmetische Zubereitung nach einem der vorhergehenden Ansprüche, **dadurch gekennzeichnet, dass** die Zubereitung frei ist von Parabenen, Phenoxyethanol, Benzethoniumchlorid, Piroctone Olamine, Lauroylarginat, Benzoesäure, Sorbinsäure, Methylisothiazolinon, Chlormethylisothiazolinon, Bronopolm Benzalkoniumchloride, Formaldehydabspalter, Salicylsäure, Triclosan, Dehydroacetsäure , DMDM Hydantoin, Chlorphenesin, IPBC.

10. Kosmetische Zubereitung nach einem der vorhergehenden Ansprüche, **dadurch gekennzeichnet, dass** die Zubereitung Ethanol enthält.

11. Kosmetische Zubereitung nach einem der vorhergehenden Ansprüche, **dadurch gekennzeichnet, dass** die Zusammensetzung einen oder mehrere weitere UV-Filter gewählt aus der Gruppe der Verbindungen Phenylen-1,4-bis-(2-benzimidazyl)-3,3-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy)disiloxa-nyl]propyl]-phenol; Terephthalidendicampher-sulfonsäure; 4-(Dimethylamino)-benzoesäure(2-ethylhexyl)ester; 4-(Dimethylamino)-benzoesäureamylester; 4-Methoxybenzalmalon-säuredi(2-ethylhexyl)ester; 4-Methoxyzimtsäureisoamylester; 2-(4'-Diethylamino-2'-hydoxybenzoyl)-benzoesäurehexylester, 2-Ethylhexyl-2-hydroxy-benzoat; Dimethicodiethylbenzalmalonat; 4-(tert.-Butyl)-4'-methoxydibenzoylmethan; 3-(4-(2,2-bis Ethoxycarbonylvinyl)-phenoxy)propenyl)-methoxysiloxan / Dimethylsiloxan - Copolymer; Dioctylbutylamido-triazon (INCI: Diethylhexyl-Butamidotriazone); 2,4-bis-[5-1(di-methylpropyl)benz-oxazol-2-yl-(4-phenyl)-imino]-6-(2-ethylhexyl)-imino-1,3,5-triazin mit der (CAS Nr. 288254-16-0); Zinkoxid, Titandioxid; 2,4,6-Tribiphenyl-4-yl-1,3,5-triazin; Merocyanine.

12. Kosmetische Zubereitung nach einem der vorhergehenden Ansprüche, **dadurch gekennzeichnet, dass** die Zubereitung 4-(tert.-Butyl)-4'-methoxydibenzoylmethan enthält.

13. Kosmetische Zubereitung nach einem der vorhergehenden Ansprüche, **dadurch gekennzeichnet, dass** die Zubereitung in Form einer O/W-Emulsion vorliegt.

14. Kosmetische Zubereitung nach einem der vorhergehenden Ansprüche, **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate Natriumstearylglutamat, enthält.

## Claims

1. Cosmetic preparation comprising
a) 2,4-bis-{[4-(2-ethylhexyloxy)-2-hydroxy]phenyl}-6-(4-methoxyphenyl)-1,3,5-triazine and/or tris(2-ethylhexyl) 4,4',4"-(1,3,5-triazine-2,4,6-triyltriimino)trisbenzoate and/or dioctylbutylamidotriazone (INCI: Diethylhexyl Butamidotriazone),
b) 2-phenylethyl benzoate,
c) di-n-butyl adipate,
**characterized in that** the preparation is free of polyethylene glycol, polyethylene glycol ethers and polyethylene glycol esters.

2. Cosmetic preparation according to Claim 1, **characterized in that** the preparation is free of N-lauroylisopropyl sarcosinate, ethylhexyl salicylate, 2-ethylhexyl 2-cyano-3,3-diphenylacrylate, homomenthyl salicylate and 2-ethylhexyl 4-methoxycinnamate and 3-(4-methylbenzylidene)camphor.

3. Cosmetic preparation according to either of the preceding claims, **characterized in that** the preparation comprises 2-phenylethyl benzoate at a concentration of 1 to 20% by weight, based on the total weight of the preparation.

4. Cosmetic preparation according to any of the preceding claims, **characterized in that** the preparation comprises di-n-butyl adipate at a concentration of 0.5 to 10% by weight, based on the total weight of the preparation.

5. Cosmetic preparation according to any of the preceding claims, **characterized in that** the ratio by weight of 2-phenylethyl benzoate to di-n-butyl adipate is from 5:1 to 1:5.

6. Cosmetic preparation according to any of the preceding claims, **characterized in that** the preparation comprises 2,4-bis-{[4-(2-ethylhexyloxy)-2-hydroxy]phenyl}-6-(4-methoxyphenyl)-1,3,5-triazine and/or tris(2-ethylhexyl) 4,4',4"-(1,3,5-triazine-2,4,6-triyltriimino)trisbenzoate and/or Diethylhexyl Butamidotriazine in a total amount of 0.1 to 15% by weight, based on the total weight of the preparation.

7. Cosmetic preparation according to any of the preceding claims, **characterized in that** the preparation comprises one or more active ingredients selected from the group of compounds comprising glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, β-alanine and/or licochalcone A, tocopherol, tocopherol acetate, Glycyrrhiza inflata root extract.

8. Cosmetic preparation according to any of the preceding claims, **characterized in that** the preparation comprises ethylhexylglycerin, propylene glycol, butylene glycol, 2-methylpropane-1,3-diol, 1,2-pentanediol, 1,2-hexanediol, 1,2-octanediol and/or 1,2-decanediol.

9. Cosmetic preparation according to any of the preceding claims, **characterized in that** the preparation is free of parabens, phenoxyethanol, benzethonium chloride, piroctone olamine, lauroyl arginate, benzoic acid, sorbic acid, methylisothiazolinone, chloromethylisothiazolinone, bronopol benzalkonium chlorides, formaldehyde releasers, salicylic acid, triclosan, dehydroacetic acid, DMDM hydantoin, chlorophenesin, IPBC.

10. Cosmetic preparation according to any of the preceding claims, **characterized in that** the preparation comprises ethanol.

11. Cosmetic preparation according to any of the preceding claims, **characterized in that** the composition comprises one or more further UV filters selected from the group of compounds comprising phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; isoamyl 4-methoxycinnamate; hexyl 2-(4'-diethylamino-2'-hydroxybenzoyl)benzoate; 2-ethylhexyl 2-hydroxybenzoate; dimethicodiethylbenzalmalonate; 4-(tert-butyl)-4'-methoxydibenzoylmethane; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); zinc oxide; titanium oxide; 2,4,6-tribiphenyl-4-yl-1,3,5-triazine; merocyanine.

12. Cosmetic preparation according to any of the preceding claims, **characterized in that** the preparation comprises 4-(tert-butyl)-4'-methoxydibenzoylmethane.

13. Cosmetic preparation according to any of the preceding claims, **characterized in that** the preparation is in the form of an O/W emulsion.

14. Cosmetic preparation according to any of the preceding claims, **characterized in that** the preparation comprises one or more O/W emulsifiers selected from the group of compounds comprising glyceryl stearate citrate, glyceryl stearate (self-emulsifying), stearic acid, stearate salts, polyglyceryl-3 methylglucose distearate, sodium cetearyl sulphate, potassium cetyl phosphate, polyglyceryl-10 stearate, sodium stearoyl glutamate.

## Revendications

1. Préparation cosmétique contenant :
a) de la 2,4-bis-{[4-(2-éthyl-hexyloxy)-2-hydroxy]-phényl}-6-(4-méthoxyphényl)-1,3,5-triazine et/ou de l'ester tris(2-éthylhexylique) de l'acide 4,4',4"-(1,3,5-triazine-2,4,6-triyltriimino)-tris-benzoïque et/ou la dioctylbutylamidotriazone (INCI : Diethylhexyl-Butamidotriazone),
b) du benzoate de 2-phényléthyle,
c) de l'adipate de di-n-butyle,
**caractérisée en ce que** la préparation est exempte de polyéthylène glycol, d'éthers de polyéthylène glycol et d'esters de polyéthylène glycol.

2. Préparation cosmétique selon la revendication 1, **caractérisée en ce que** la préparation est exempte de sarcosinate de N-lauroylisopropyle, de salicylate d'éthylhexyle, d'acrylate de 2-éthylhexyl-2-cyano-3,3-diphényle, de salicylate d'homomenthyle et d'ester 2-éthylhexylique de l'acide 4-méthoxycinnamique et de 3-(4-méthylbenzylidène)camphre.

3. Préparation cosmétique selon l'une quelconque des revendications précédentes, **caractérisée en ce que** la préparation contient du benzoate de 2-phényléthyle en une concentration de 1 à 20 % en poids, par rapport au poids total de la préparation.

4. Préparation cosmétique selon l'une quelconque des revendications précédentes, **caractérisée en ce que** la préparation contient de l'adipate de di-n-butyle en une concentration de 0,5 à 10 % en poids, par rapport au poids total de la préparation.

5. Préparation cosmétique selon l'une quelconque des revendications précédentes, **caractérisée en ce que** le rapport en poids entre le benzoate de 2-phényléthyle et l'adipate de di-n-butyle est de 5:1 à 1:5.

6. Préparation cosmétique selon l'une quelconque des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine et/ou de l'ester tris(2-éthylhexylique) de l'acide 4,4',4"-(1,3,5-triazine-2,4,6-triyltrümino)-tris-benzoïque et/ou la diéthylhexylbutamidotriazine en une quantité totale de 0,1 à 15 % en poids, par rapport au poids total de la préparation.

7. Préparation cosmétique selon l'une quelconque des revendications précédentes, **caractérisée en ce que** la préparation contient un ou plusieurs agents actifs choisis dans le groupe constitué par les composés acide gylcyrrhizique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, β-alanine et/ou licochalcone A, tocophérol, acétate de tocophérol, Glycyrrhiza Inflata Root Extract.

8. Préparation cosmétique selon l'une quelconque des revendications précédentes, **caractérisée en ce que** la préparation contient de l'éthylhexylglycérine, du propylène glycol, du butylène glycol, du 2-méthylpropane-1,3-diol, du 1,2-pentanediol, du 1,2-hexanediol, du 1,2-octanediol et/ou du 1,2-décanediol.

9. Préparation cosmétique selon l'une quelconque des revendications précédentes, **caractérisée en ce que** la préparation est exempte de parabènes, de phénoxyéthanol, de chlorure de benzéthonium, de piroctone olamine, d'arginate de lauroyle, d'acide benzoïque, d'acide sorbique, de méthylisothiazolinone, de chlorométhylisothiazolinone, de chlorure de bromopolm benzalkonium, de cliveurs de formaldéhyde, d'acide salicylique, de triclosan, d'acide déshydroacétique, de DMDM hydantoïne, de chlorphénésine, d'IPBC.

10. Préparation cosmétique selon l'une quelconque des revendications précédentes, **caractérisée en ce que** la préparation contient de l'éthanol.

11. Préparation cosmétique selon l'une quelconque des revendications précédentes, **caractérisée en ce que** la composition contient un ou plusieurs filtres UV supplémentaires choisis dans le groupe constitué par les composés sels de l'acide phénylène-1,4-bis-(2-benzimidazyl)-3,3-5,5'-tétrasulfonique ; sels de l'acide 2-phénylbenzimidazole-5-sulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; acide téréphtalidène-dicamphre-sulfonique ; ester 2-éthylhexylique de l'acide 4-(diméthylamino)-benzoïque ; ester amylique de l'acide 4-(diméthylamino)-benzoïque ; ester di(2-éthylhexylique) de l'acide 4-méthoxybenzalmalonique ; ester isoamylique de l'acide 4-méthoxycinnamique ; ester hexylique de l'acide 2-(4'-diéthylamino-2'-hydroxybenzoyl)-benzoïque, 2-hydroxy-benzoate de 2-éthylhexyle ; benzalmalonate de diméthicodiéthyle ; 4-(tert.-butyl)-4'-méthoxydibenzoylméthane ; copolymère de 3-(4-(2,2-bis éthoxycarbonylvinyl)-phénoxy)propényl)-méthoxysiloxane/diméthylsiloxane ; dioctylbutylamido-triazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine de n°CAS 288254-16-0 ; oxyde de zinc, dioxyde de titane ; 2,4,6-tribiphényl-4-yl-1,3,5-triazine ; mérocyanine.

12. Préparation cosmétique selon l'une quelconque des revendications précédentes, **caractérisée en ce que** la préparation contient du 4-(tert.-butyl)-4'-méthoxydibenzoylméthane.

13. Préparation cosmétique selon l'une quelconque des revendications précédentes, **caractérisée en ce que** la préparation se présente sous la forme d'une émulsion H/E.

14. Préparation cosmétique selon l'une quelconque des revendications précédentes, **caractérisée en ce que** la préparation contient un ou plusieurs émulsifiants H/E choisis dans le groupe constitué par les composés stéarate-citrate de glycéryle, stéarate de glycéryle (auto-émulsifiant), acide stéarique, sels de stéarate, distéarate de polyglycéryl-3-méthylglycose, sulfate de cétéaryle de sodium, phosphate de cétyle de potassium, stéarate de polyglycéryl-10 glutamate de stéaryle de sodium.
